(19) Europäisches Patentamt
European Patent Office
Office européen des brevets

(11) Veröffentlichungsnummer: **0 001 075**
**B1**

(12) **EUROPÄISCHE PATENTSCHRIFT**

(21) Anmeldenummer: **78100747.1**

(22) Anmeldetag: **25.08.78**

(51) Int. Cl.³: **C 07 C 103/52,**
A 61 K 37/02

# (54) Teilgeschützte Human-Insulin-A-Kette und Verfahren zu ihrer Herstellung

(30) Priorität: **08.09.77 DE 2740406**

(43) Veröffentlichungstag der Anmeldung:
**21.03.79 Patentblatt 79/06**

(45) Bekanntmachung des Hinweises auf die Patenterteilung:
**26.11.80 Patentblatt 80/24**

(84) Benannte Vertragsstaaten:
**BE CH DE FR GB**

(56) Entgegenhaltungen:
**FR - A - 2 060 002**
**NL - A - 7 406 840**

(73) Patentinhaber: **HOECHST AKTIENGESELLSCHAFT**
**Postfach 80 03 20**
**D - 6230 Frankfurt/Main 80 (DE)**

(72) Erfinder: **König, Wolfgang, Dr.**
**Eppsteiner Strasse 25**
**D - 6238 Hofheim am Taunus (DE)**

**EP 0 001 075 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Teilgeschützte Human-Insulin-A-Kette und Verfahren zu ihrer Herstellung

Die Synthese von Insulinen, ausgehend von den beiden Peptidketten A- bzw. B-Kette wird besonders vorteilhaft mit Hilfe von Brückenreagenzien die die $\alpha$-Aminogruppe in A 1 mit der $\varepsilon$-Aminogruppe in B 29 verknüpfen, durchgeführt (Biochem. Biophys. Comm. *55,* 60—66 (1973)). Die anschließend Oxydation der Mercaptogruppen zu Disulfiden und Abspaltung des Brückenreagenzes ergibt eine wesentlich bessere Ausbeute an Insulin als die Oxydation isoliert vorliegender Ketten. Um die Ketten gezielt verknüpfen zu können, müssen sie in geeignet geschützter Form vorliegen.

Die Sequenz der Human-Insulin-A-Kette wurde gemäß J. Am. Chem. Soc. 89, 4505 (1967) bereits synthetisch aufgebaut; dieses Peptid ist jedoch für die Kupplung mit Brückenreagentien weniger geeignet.

Gegenstand der Erfindung ist eine partiell geschützte Humaninsilin-A-Kette der Formel I

H-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)Gln-Leu-Glu(OBu$^t$)-Asn-Tyr(Bu$^t$)-Cys-(Trt)-Asn-OBu$^t$

Gegenstand der Erfindung ist weiterhin ein Verfahren zur Herstellung des Peptides der Formel I, das dadurch gekennzeichnet ist, daß man das Peptid der Formel II

H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

mit dem Peptid der Formel III

X-Gly-Ile-Val-Glu(OBu$^t$)-Gln-OH

worin X eine Aminoschutzgruppe bedeutet, die durch 5% Trifluoressigsäure in 95% Methylenchlorid abspaltbar ist, nach der Dicyclohexylcarbodiimid-Methode unter Zusatz von N-Hydroxyverbindungen kondensiert und die N-terminale Schutzgruppe X aus dem erhaltenen Peptid selektiv mit ca. 5%iger Trifluoressigsäure in Methylenchlorid abspaltet.

Als N-Hydroxyverbindung als Zusatz bei der Kondensation mit Dicyclohexylcarbodiimid (DCC) kommt insbesondere 1-Hydroxybenzotriazol (HOBt) in Betracht. Anstelle von HOBt können jedoch auch N-Hydroxysuccinimid, 3-Hydroxy-4-oxo-3,4-dihydro-1,2,3-benzotriazin oder 2-Hydroxy-imino-2-cyano-essigsäureäthylester verwendet werden. Die Kupplungsreaktionen werden bei 0°C bis Raumtemperatur in polaren Lösungsmitteln wie z.B. Dimethylformamid oder Dimethylacetamid, sowie in Mischungen dieser Lösungsmittel durchgeführt. Als verwendbare N-terminale Schutzgruppe X kommen in Betracht die 3,5-Dimethoxy-$\alpha,\alpha$-dimethylbenzyloxycarbonylgruppe (Ddz) die Diphenyl-isopropyloxycarbonylgruppe (Bpoc) sowie die Tritylgruppe.

Die selektive Abspaltung dieser Schutzgruppe X wird in ungefähr 5%iger Trifluoressigsäure-Methylenchlorid-Lösung durchgeführt, der auch etwas Wasser (ca. 1%) und Anisol (ca. 10%) oder ähnlich wirkende Verbindungen zugesetzt werden kann.

Die Herstellung des Peptides der Formel II und dessen weitere Umsetzung nach dem erfindungsgemäßen Verfahren zum Peptide der Formel I kann nach dem Reaktionsschema I erfolgen. Hierbei wird in allen Vorstufen bevorzugt die Ddz-Gruppe als Schutzgruppe am Aminoende verwendet, nach deren Entfernung die Kondensation mit dem nächsten Ddz-Peptid erfolgt.

Bei der Synthese der Vorstufen eignet sich die Ddz-Gruppe, die aus aus Annalen der Chemie 763, S. 162 bis 172 (1972) bereits bekannt ist, in besonderer Weise, da sie einerseits in schwach saurer Lösung leicht abspaltbar ist, andererseits aber überraschenderweise gegenüber katalytischer Hydrierung stabil ist. So können z.B. Benzyl oder Nitrobenzylester selektiv durch katalytische Hydrierung in Anwesenheit der Ddz-Gruppe gespalten werden.

Reaktionsschema I:

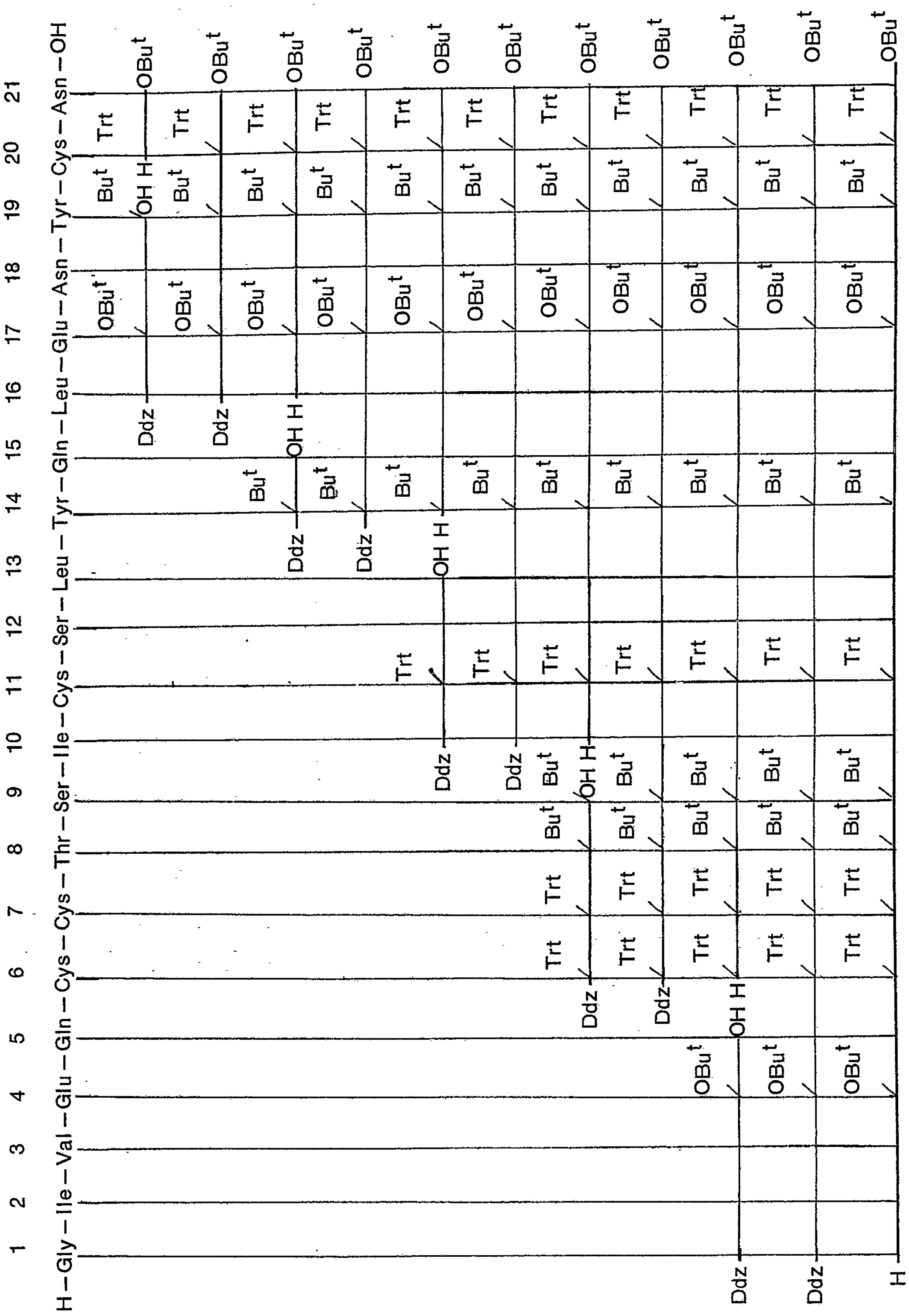

Die einzelnen, nach Schema I benötigten Peptidbruchstücke werden wie folgt hergestellt:

H-Cys(Trt)-Asn-OBu$^t$ (Fragment 20—21) wurde analog Helv. Chim. Acta *54*, 398—422 (1971) hergestellt:

Ddz-Leu-Glu(OBu$^t$)-Asn-Tyr(Bu$^t$)-OH (Fragment 16—19) wurde nach Reaktionsschema II durch schrittweisen Aufbau mit Z- bzw. Ddz-Aminosäure-2,4,5-trichlorphenylester unter HOBt-Katalyse aufgebaut. Die intermediären Z-Schutzgruppen werden durch katalytische Hydrierung entfernt.

Reaktionsschema II:

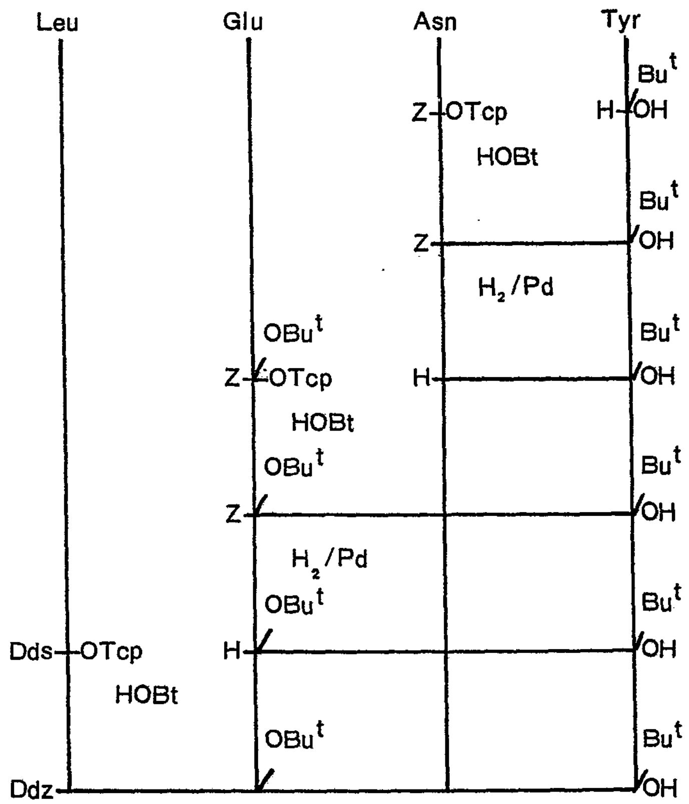


Ddz-Tyr(Bu$^t$)-Gln-OH (Fragment 14—15) wurde durch katalytische Hydrierung von Ddz-Tyr(Bu$^t$)-Gln-ONb gewonnen.

Ddz-Ile-Cys(Trt)-Ser-Leu-OH (Fragment 10—13) wird durch Umsetzung von Ddz-Ile-OTcp mit dem bereits bekannten H-Cys(Trt)-Ser-Leu-OH (Chem. Ber. *103*, 2034—2040 (1970)) erhalten.

Ddz-Cys(Trt)-Cys(Trt)-Thr(Bu$^t$)-Ser-(Bu$^t$)-OH (Fragment 6—9) wird analog Reaktionsschema III synthetisiert.

Reaktionsschema III:

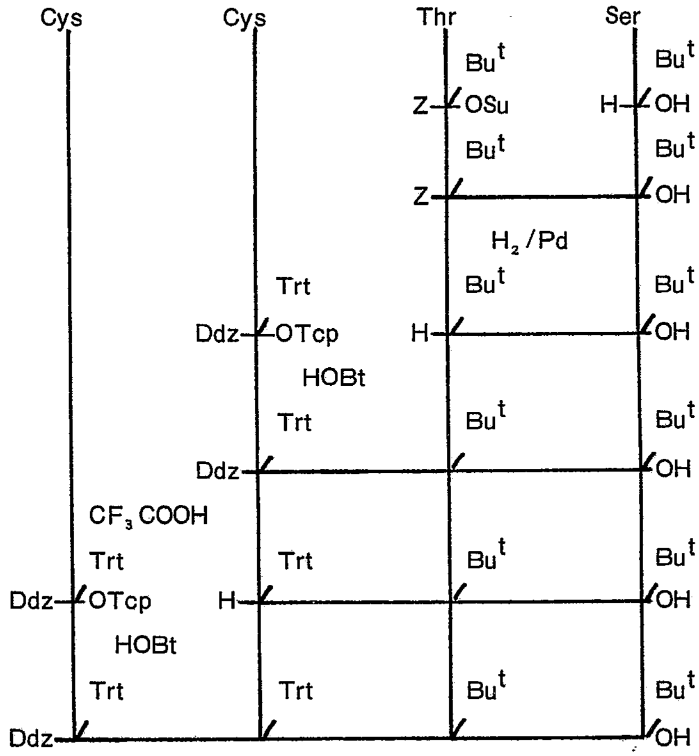


Die andere Komponente nach dem erfindungsgemäßen Verfahren, das Fragment 1—5 der Formel III Ddz-Gly-Ile-Val-Glu(OBu$^t$)-Gln-OH kann nach zwei verschiedenen Methoden (Schema IV oder V) hergestellt werden.

Reaktionsschema IV:

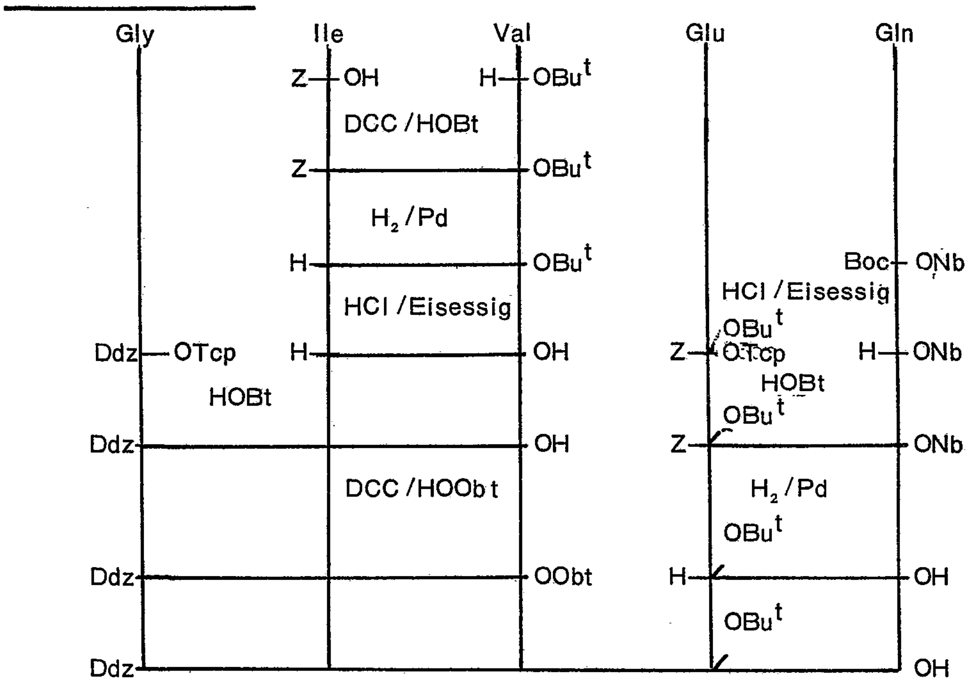

Reaktionsschema V:

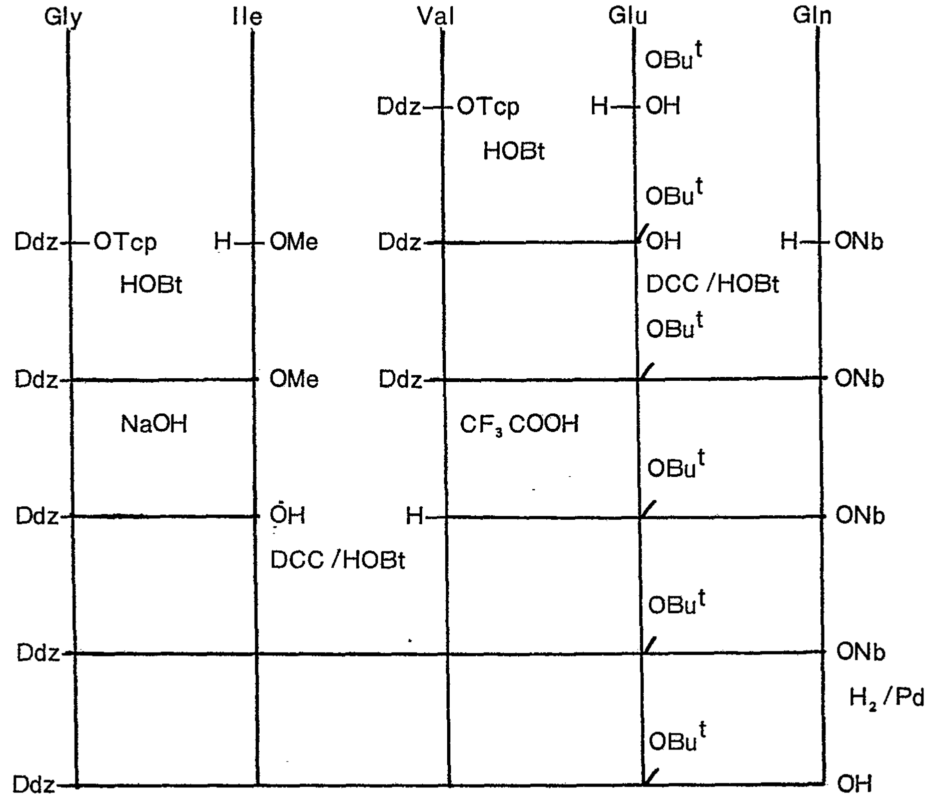


Beispiel

*I. Synthese von Fragment 16—19*

Ddz-Leu-Glu(OBu$^t$)-Asn-Tyr(Bu$^t$)-OH

*1. Ddz-Leu-OTcp*

33,3 g Ddz-Leu-OH (Ann. Chem. *1973*, 1652—1662), 94,3 mMol) werden mit 19,6 g (0,1 Mol) 2,4,5-Trichlorphenol in 350 ml Essigester gelöst. Dazu gibt man bei 0°C eine Lösung von 20,4 g DCC in 60 ml Essigester. Man läßt 3 Stdn. bei 0°C und über Nacht bei Raumtemperatur rühren. Der ausgefallene Dicyclohexylharnstoff wird abgesaugt und das Filtrat eingeengt. Der Rückstand kristallisiert mit Petroläther über Nacht im Kühlraum.

Ausbeute 39,2 g (73,6% bezogen auf H-Leu-OMe.HCl).
Schmp. 108°, $[\alpha]_D^{29} = -31{,}3°$ (c = 1, in Dimethylacetamid)

| | | | | | |
|---|---|---|---|---|---|
| $C_{24}H_{28}NO_6Cl_3$ (532,83) | Ber. | C 54,10 | H 5,30 | N 2,62 | Cl 20,01 |
| | Gef. | C 54,7 | H 5,4 | N 2,9 | Cl 19,3 |

*2. Ddz-Leu-Glu(OBu$^t$)-Asn-Tyr(Bu$^t$)-OH.0,5$H_2O$*

27,7 g (50 mMol) H-Glu(OBu$^t$)-Asn-Tyr(Bu$^t$)-OH (Chem. Ber. *110*, 1—11 (1977)) werden in 150 ml Dimethylformamid suspendiert. Nach Zugabe von 6,75 g HOBt (50 mMol) tritt Lösung ein. Man kühlt auf 0°C ab und gibt 6,5 ml (50 mMol) N-Äthylmorpholin und 28 g (52,5 mMol) Ddz-Leu-OTcp zu. Es wird 1 Stdn. bei 0°C und 3 Stdn. bei Raumtemperatur gerührt. Eine Fluoram-probe ergab, daß noch nicht die ganze Aminokomponente abreagiert war. Man gibt nun nochmals 1,3 g Ddz-Leu-OTcp (2,5 mMol) zu und läßt einige Stunden bei Raumtemperatur reagieren. Ist der Ansatz Fluoramnegative, wird aufgearbeitet.

Das Lösungsmittel wird im Hockvakuum abdestilliert und der Rückstand in Essigester gelöst. Es wird mit eiskaltem Citratpuffer (pH 3) auf pH 4 eingestellt. Anschließend wird die Essigesterphase einmal mit Citratpuffer (pH 4) und zwei Mal mit Wasser gewaschen. Man trocknet über $Na_2SO_4$ und engt ein. Der Rückstand wird mit einer Mischung aus Äther und Petroläther wie 1:1 verrieben. Man stellt einige Stunden in den Kühlraum und saugt ab.

Ausb. 35,35 g (80,25%).
Schmp. 222—223°, $[\alpha]_D^{25} = -23,7°$ (c = 1, in Methanol)

$C_{44}H_{65}N_5O_{13}$.0,5 $H_2$ (881,04) Ber. C 60,00 H 7,55 N 7,95
Gef. C 60,0 H 7,6 N 8,0

*II. Synthese von Fragment 14—15:*
Ddz-Tyr(Bu$^t$)-Gln-OH

*1. H-Tyr(Bu$^t$)-OMe.HCl*

300 g (778 mMol) Z-Tyr(Bu$^t$)-OMe (Ann. Chem. *696*, 226 (1966)) werden in 1 ltr. Methanol gelöst und an der Autobürette (Zugabe von ca 2n methanolischer HCl) bei pH 4,5 katalytisch (Pd/$BaSO_4$-Katalysator) hydriert. Nach beendigter Hydrierung wird der Katalysator abgesaugt und das Filtrat eingeengt. Der Rückstand wird mit Äther verrieben.

Ausb. 189,2 g (84,5%).
Schmp. 154—156°, $[\alpha]_D^{22} = +15,0°$ (c = 1, in Methanol)

$C_{14}H_{22}NO_3Cl$ (287,79) Ber. C 58,43 H 7,7 N 4,86
Gef. C 58,6 H 7,6 N 4,8

*2. Ddz-Tyr(Bu$^t$)-OMe*

Zu einer Lösung von 71,95 g (0,25 Mol) H-Tyr(Bu$^t$)-OMe. HCl in 110 ml Dimethylformamid gibt man unter Eiskühlung und Rühren 35 ml (0,25 Mol) Triäthylamin und 79,5 (0,3 Mol) Ddz-azid. Nach 5 Minuten gibt man weitere 35 ml (0,25 Mol) Triäthylamin zu. Nun entfernt man das Eisbad und läßt auf Raumtemperatur kommen. Nach 30 Minuten gibt man 7 ml (50 mMol) Triäthylamin zu und läßt 3 Stunden bei Raumtemperatur rühren. Der Ansatz bleibt über Nacht bei Raumtemperatur stehen, und wird zwischen 250 ml Essigester und 250 ml Wasser verteilt. Die Essigesterphase wird nach Zugabe von Eis 3 mal mit je 125 ml eiskaltem Citratpuffer (pH 3), 1 mal mit 250 ml ges. $NaHCO_3$-Lösung und 1 mal mit 125 ml Wasser gewaschen, über $Na_2SO_4$ getrocknet und eingeengt. Am Hochvakuum wird getrocknet. Ausb. 118,2 g Ol (99,8%).

*3. Ddz-Tyr(Bu$^t$)-OH*

Die unter 2) gewonnenen 118,2 g (249,5 mMol) öligen Ddz-Tyr(Bu$^t$)-OMe werden in 500 ml 1,2-Dimethoxyäthan gelöst und mit einer Spatelspitze Thymolphalein versetzt. Dazu läßt man unter Rühren 250 ml 1n NaOH zutropfen. Man rührt bis die blaue Farbe des Ansatzes verblaßt ist. Nun werden nach und nach in mehreren Portionen noch 120 ml 1n NaOH zugesetzt bis sich die Lösung nicht mehr entfärbt (Der Mehrverbrauch an Natronlauge erklärt sich durch den Essigestergehalt des Ausgangsproduktes). Danach wird mit Citronensäure neutralisiert und die Lösung eingeengt.

Der Rückstand wird mit Eiswasser versetzt und bei 0°C mit Citronensäure auf pH 3,5 gestellt. Das entstehende Ol wird in Essigester aufgenommen. Die Essigesterphase wird zwei mal mit 200 ml Citratpuffer (pH 4) und zwei mal mit 200 ml Wasser ausgeschüttelt. Die Essigesterphase wird über $Na_2SO_4$ getrocknet, abdestilliert und am Hochvakuum getrocknet. Es bleibt eine gelblich-weiße amorphe Substanz zurück.

Ausb. 99,5 g (86,8%).
Schmp. ca. 38—40°, $[\alpha]_D^{22} = +41,5°$ (c = 1, in Methylenchlorid).

$C_{25}H_{33}NO_7$ (459,55) Ber. C 65,34 H 7,24 N 3,05
Gef. C 65,0 H 7,3 N 3,0

*4. Ddz-Tyr(Bu$^t$)-Gln-ONb*

Zu einer Lösung von 25,24 g (55 mMol) Ddz-Tyr(Bu$^t$)-OH 15,88 g (50 mMol) H-Gln-ONb.HCl (Chem. ber. *110*, 1—11 (1977)) und 6,75 g HOBt monohydrat in 100 ml Dimethylformamid gibt man bei 3°C 6,4 ml (50 mMol) N-Athylmorpholin und 10,5 g DCC. Man läßt 1 Stdn. bei 0°C und 6 Stdn. bei Raumtemperatur rühren und über Nacht bei Raumtemperatur stehen. Der Niederschlag wird abgesaugt und das Filtrat eingeengt. Das resultierende Öl wird in Essigester gelöst und die Lösung nacheinander mit $NaHCO_3$-Lösung, Citratpuffer (pH 3) und Wasser gewaschen, über $Na_2SO_4$ getrocknet und eingeengt. Das Öl wird mit Petroläther pulvrig verrieben und abgesaugt. Zur weiteren Reinigung wird die Substanz drei mal mit je 100 ml Diisopropyläther gekocht und abdekantiert. Zum Schluß wird mit kaltem Diisopropyläther verrieben, abgesaugt und mit Petroläther nachgewaschen.

Ausb. 32,8 g (91%).
Schmp. 80—90° $[\alpha]_D^{22} = -15,1°$ (c = 1, in Methanol)

$C_{37}H_{46}N_4O_{11}$ (722,81) Ber. C 61,48 H 6,41 N 7,75
Gef. C 61,3 H 6,7 N 7,9

*5. Ddz-Tyr(Bu^t)-Gln-OH-DCHA*

Zu einer Lösung von 32,5 g (45 mMol) Ddz-Tyr(Bu^t)-Gln-ONb in 500 ml Methanol gibt man 5 ml Wasser und Pd/$BaSO_4$-Katalysator und hydriert 7 Stunden. Danach wird vom Katalysator abgesaugt und das Filtrat eingeengt. Das zurückbleibende Öl wird in 250 ml Essigester gelöst. Dazu gibt man 11,3 ml (55 mMol) Dicyclohexylamin. Man läßt einige Stunden im Kühlraum stehen und saugt den Niederschlag ab. Der Filterrückstand wird mit Essigester in der Reibeschale verrieben, abgesaugt und i.Vak. getrocknet.

Ausb. 27 g (78%).
Schmp. 170—171°, $[\alpha]_D^{23}$ = +10,2° (c = 1, in Methanol).

$C_{42}H_{64}N_4O_9$ (769,0) Ber. C 65,6 H 8,39 N 7,28
Gef. C 65,4 H 8,5 N 7,3

*6. Ddz.-Tyr(Bu^t)-Gln-OH*

2,9 g (3,77 mMol) Ddz-Tyr(Bu^t)-Gln-OH-DCHA werden zwischen Essigester und Citratpuffer (pH 3) verteilt. Die Essigesterphase wird mit Wasser neutral gewaschen, über $Na_2SO_4$ getrocknet und eingeengt. Es bleibt ein amorphes Produkt zurück.

Ausb. 2 g (90%).
Schmp. 110—115°C, $[\alpha]_D^{22}$ = +19,8° (c = 1, in Methanol).

$C_{30}H_{41}N_3O_9$ (587,68) Ber. C 61,31 H 7,03 N 7,15
Gef. C 60,6 H 7,2 N 7,0

*III. Synthese von Fragment 10—13:*
Ddz-Ile-Cys(Trt)-Ser-Leu-OH

*1. Ddz-Ile-OTcp*

Zu einer auf 0°C gekühlten Lösung von 87 g (246 mMol) Ddz-Ile-OH (Ann. Chem. *763,* 162—172 (1072)) und 48,6 g (246 mMol) 2,4,5-Trichlorphenol in 700 ml Essigester gibt man eine kalte Lösung von 52 g (252 mMol) DCCl in 175 ml Essigester. Man läßt 3 Stunden bei 0°C rühren und über Nacht bei Raumtemperatur stehen. Der Dicyclohexylharnstoff wird abgesaugt und das Filtrat i. Vak. eingeengt. Der Rückstand kristallisiert mit Petroläther.

Ausb. 105,6 g (80,5%).
Schmp. 92—94°, $[\alpha]_D^{22}$ = −22,6° (c = 1, in Methanol).

$C_{24}H_{28}NO_6Cl_3$ (532,86) Ber. C 54,10 H 5,30 N 2,63 Cl 19,96
Gef. C 54,4 H 5,4 N 2,8 Cl 19,5

*2. Ddz-Ile-Cys(Trt)-Ser-Leu-OH.1 $H_2O$*

Zu einer Lösung von 69,8 g (120 mMol) H-Cys(Trt)-Ser-Leu-OH-monohydrat (Chem. Ber. 103, 2034—2040 (1970)) und 16,2 g (120 mMol) HOBt in 300 ml Dimethylformamid gibt man bei Raumtemperatur 63,95 g (120 mMol) Ddz-Ile-OTcp und rührt 4 Stunden bei Raumtemperatur. Danach gibt man nochmals 5,33 g (10 mMol) Ddz-Ile-OTcp zu, wenn Tripeptid noch vorhanden ist. Man läßt über Nacht bei Raumtemperatur stehen und destilliert das Lösungsmittel im Vakuum ab. Das zurückbleibende Öl wird zwischen 400 ml Essigester und 250 ml gesättigter Natriumbicaronatlösung verteilt, wobei das Natriumsalz des Ddz-Tetrapeptids im Essigester bleibt. Die Essigesterphase wird gekühlt und nacheinander mit ca. 120 ml 1n Citronensäurelösung (wässrige Phase sollte nach dem Ausschütteln etwa pH 3—4 haben), 150 ml Citratpuffer (pH 3) und 2 mal mit 200 ml Wasser ausgeschüttelt. Danach wird die Essigesterphase über $Na_2SO_4$ getrocknet und im Vakuum eingeengt. Der Rückstand wird mit Petroläther verrieben und abgesaugt.

Ausb. 99,8 (90%).
Schmp. (130) 135—140° (Zers.) $[\alpha]_D^{22}$ = 34,1° (c = 1, in Methanol).

$C_{49}H_{62}N_4O_{10}S.1\ H_2O$ (917,1) Ber. C 62,93 H 7,11 N 6,02 S 3,42
Gef. C 63,0 H 6,8 N 5,8 S 3,4

*IV. Synthese von Fragment 6—9:*
Ddz-Cys(Trt)/-Cys(Trt)-Thr(Bu^t)-Ser(Bu^t)-OH

*1. Z.Thr(Bu^t)-Ser(Bu^t)-OH*

Zu einer Suspension von 16,1 g (0,1 mol) H-Ser(Bu^t)-OH (Chem. Ber. *97,* 2490 (1964) in 100 ml

Dimethylformamid gibt man bei 0°C 12,8 ml (0,1 mol) N-Athylmorpholin und 40,6 g (0,1 mol) Z-Thr(Bu[t])-OSu (Hoppe Seyler's Z. Physiol. Chem. *346*, 60(1966)). Man rührt anschließend 5 Stunden bei Raumtemperatur und läßt über Nacht bei Raumtemperatur stehen.

DC-Kontrolle auf H-Ser(Bu[t])-OH!

Ist kein H-Ser(Bu[t])-OH dünnschichtchromatographisch feststellbar, wird von gegebenenfalls Ungelöstem abgesaugt. Das Filtrat wird eingeengt und der Rückstand zwischen Äther und 110 ml 1n Zitronensäure verteilt. Die ätherische Phase wird noch einmal mit 100 ml 0,5 n Zitronensäure und anschließend mit Wasser oder NaCl-Lösung neutral gewaschen. Die ätherische Phase wird über $Na_2SO_4$ getrocknet, eingeengt und am Hochvakuum getrocknet.

Ausb. 32,5 g amorphes Produkt (72%) $[\alpha]_D^{22} = +24,1°$ (c = 1, in Methanol).

$C_{22}H_{36}N_2O_7$ (452,55) Ber. C 61,04 H 8,02 N 6,19
Gef. C 60,3 H 7,4 N 6,2

*2. H-Thr(Bu[t])-Ser(Bu[t])-OH.HCl*

25 g (55,24 mMol) Z-Thr(Bu[t])-Ser(Bu[t])OH werden in 200 ml Methanol gelöst und am Autotitrator unter Zugabe von methanol. HCl katalytisch hydriert (pH 4,5). Nach beendigter Hydrierung wird von $Pd/BaSO_4$-Katalysator abgesaugt und das Filtrat eingeengt und am Hochvakuum getrocknet.

Ausb. 21,1 g amorphes Produkt (19,60 = 100%) $[\alpha]_D^{27} = +27°$ (c = 1, in Methanol).

$C_{15}H_{31}N_2O_5Cl$ (354,9) Ber. C 50, 77 H 8,80 N 7,89
Gef. C 50,9 H 8,8 N 7,8

*3. Ddz-Cys-(Trt)-OH*

Zu einer Suspension von 36,4 g (0,1 mol) H-Cys(Trt)-OH (J. Org. Chem. 30, 1340 (1965)) in 200 ml Dimethylformamid gibt man 28 ml (0,2 mol) Triäthylamin und 26,5 g (0,1 mol) Ddz-azid. Es wird 24 Stunden bei 40°C gerührt. Danach wird die Lösung eingeengt und der Rückstand zwischen 100 ml eisgekühlter 1n Zitronensäure und Essigester verteilt. Die Essigesterphase wird mit Zitratpuffer (pH 3) und NaCl-Lösung ausgeschüttelt, über $Na_2SO_4$ getrocknet und eingeengt. Am Hochvakuum wird getrocknet.

Ausb. 37,6 g amorphes Produkt (64%), $[\alpha]_D^{22} = +27,0°$ (c = 1, in Methanol).

$C_{34}H_{35}NO_6S$ (585,7) Ber. C 69,72 H 6,02 N 2,39
Gef. C 69,3 H 6,3 N 2,6

*4. Ddz-Cys(Trt)-OTcp*

Zu einer Lösung von 5,9 g (ca. 10 mMol) Ddz-Cys(Trt)-OH und 2,2 g (11 mMol) 2,4,5-Trichlorphenol in 50 ml absolutem Tetrahydrofuran gibt man bei 0°C 2,3 g DCC, gelöst in 15 ml Tetrahydrofuran, rührt 2 Stunden bei 0°C und läßt über Nacht bei Raumtemperatur stehen. Der DC-Harnstoff wird abgesaugt und die Lösung eingeengt. Der Rückstand wird in Methylenchlorid gelöst und über 100 g Kieselgel 60 filtriert. Man eluiert mit Methylenchlorid.

Ausb. 6,2 g amorphes Produkt (81%).
$[\alpha]_D^{22} = +10,3°$ (c = 1, in Methanol).

$C_{40}H_{36}Cl_3NO_6S$ (765,17) Ber. C 62,79 H 4,74 N 1,83 S 4,19
Gef. C 61,0 H 4,7 N 1,7 S 4,2

Laut CHN-Analyse dürfte die Substanz 97%ig sein.

*5. Ddz-Cys(Trt)-Thr(Bu[t])-Ser(Bu[t])-OH*

Zu einer Lösung von 31,8 g (0,1 mol) H-Thr(Bu[t])-Ser(Bu[t])-OH (oder entsprechend mehr je nach Wasser oder Säuregehalt) und 13,5 g HOBt (0,1 mol) in 100 ml Dimethylformamid gibt man bei 0°C 12,8 ml (0,1 mol) N-Äthylmorpholin und 76,5 g (0,1 mol) Ddz-Cys(Trt)-OTcp. Anschließend wird ca. 6 Stunden bei Raumtemperatur gerührt und die Lösung im Hochvakuum eingeengt. Der Rückstand wird zwischen Eis-Wasser und Essigester verteilt. Die Essigesterphase wird mit $NaHCO_3$-Lösung ausgeschüttelt, wobei Ddz-Cys(Trt)-Thr(Bu[t])-Ser(Bu[t])-OH als Natriumsalz in der Essigesterphase bleibt. Danach wird bei 0°C mit 100 ml 1n Zitronensäure und 100 ml Citratpuffer (pH 3) ausgeschüttelt und mit Wasser neutral gewaschen. Die Essigesterphase wird über $Na_2SO_4$ getrocknet, eingeengt und der Rückstand im Hochvakuum getrocknet. Zur weiteren Reinigung wird die Substanz aus Äther/Petroläther umgefällt, wobei die ätherische Lösung in den vorgelegten Petroläther langsam eingetropft wird.

Ausb. 44,5 g (50%)
Schmp. 116—128° (Zers.), $[\alpha]_D^{22} = +25,5°$ (c = 1, in Methanol).

$C_{49}H_{63}N_3O_{10}S$ (886,13) Ber. C 66,42 H 7,17 N 4,74
Gef. C 66,1 H 7,4 N 4,7

*6. H-Cys(Trt)-Thr(Bu$^t$)-Ser(Bu$^t$)-OH-trifluoracetat*

8,8 g (10 mMol) Ddz-Cys(Trt)-Thr(Bu$^t$)-Ser(Bu$^t$)-OH werden bei Raumtemperatur in einer Mischung aus 8,75 ml (0,1 mol) Trifluoressigsäure, 1,75 ml Wasser und 165 ml Methylenchlorid (= ca. 175 ml 5%ige Trifluoressigsäure in Methylenchlorid mit 1% Wasser) gelöst. Man läßt 1,5 Stunden bei Raumtemperatur stehen und stellt mit 8,85 ml (0,11 mol) Pyridin neutral. Die Lösung wird eingeengt und der Rückstand zwischen Essigester und Wasser verteilt. Die Essigesterphase wird noch zwei mal mit Wasser gewaschen, über $Na_2SO_4$ getrocknet und eingeengt. Der Rückstand wird in wenig Äther gelöst und langsam in Petroläther eingetropft.

Ausb. 4,2 g (54%).
Schmp. 85—95° (Zersetzung), $[\alpha]_D^{22} = +68,5°$ (c = 1, in Methanol).

$C_{37}H_{49}N_3O_6S.CF_3COOH$ (777,9) Ber. C 60,21 H 6,48 N 5,40 S 4,12
Gef. C 60,7 H 6,7 N 5,2 S 4,2

*7. Ddz-Cys(Trt)-Cys(Trt)-Thr(Bu$^t$)-Ser(Bu$^t$)-OH*

Zu einer Lösung von 5,3 g (6,8 mmol) H-Cys(Trt)-Thr(Bu$^t$)-Ser(Bu$^t$) OH-trifluoracetat und 0,95 g (0,7 mmol) HOBt in 15 ml Dimethylformamid gibt man bei 0° 0,9 ml (0,7 mmol) N-Äthylmorpholin und 5,35 g (7 mmol) Ddz-Cys(Trt)-OTcp. Man rührt 14 Stunden bei Raumtemperatur und engt die Lösung ein, wenn das Tripeptid abreagiert ist. (Sollte noch unreagiertes Tripeptid nachweisbar sein, muß noch etwas Ddz-Cys(Trt)-OTcp zugesetzt werden). Den Rückstand nimmt man in Essigester auf. Die Essigesterphase wird nacheinander mit 10—20 ml gesättigter $NaHCO_3$-Lösung, mit 7 ml 1n Zitronensäure und 10 ml Zitratpuffer (pH 3) und mit NaCl-Lösung neutral gewaschen, über $Na_2SO_4$ getrocknet und eingeengt. Der Rückstand wird mit Petroläther verrieben. Zur weiteren Reinigung wird aus Äther-Petroläther gefällt oder mit Diisopropylätger verührt. Auch aus Methanol/Wasser kann umgefällt werden.

Ausb. 6 g (71%).
Schmp. 118—130° (Zers.), $[\alpha]_D^{22} = +11,6°$ (c = 1 in Methanol).

$C_{71}H_{82}N_4O_{11}S_2$ (1231,6) Ber. C 69,25 H 6,71 N 4,55 S 5,21
Gef. C 67,2 H 6,3 N 4,1 S 5,3

Laut CHN-Analyse ist die Substanz nur etwa 97%ig.

*V. Synthese von Fragment 1—5*

Ddz-Gly-Ile-Val-Glu(OBu$^t$)-Gln-OH

*1. Ddz-Val-OTcp*

Zu einer Lösung von 113,3 g (0,334 mmol) Ddz-Val-OH (Liebigs Ann. 763, 162—172 (1972)) und 65,9 g (0,334 mmol) 2,4,5-Trichlorphenol in 300 ml Essigester gibt man bei 0°C eine Lösung von 72,1 g (0,35 mmol) DCC in 100 ml Essigester. Man läßt 1 Stunde bei 0° und 3 Stunden bei Raumtemperatur rühren. Der DC-Harnstoff wird abgesaugt und das Filtrat eingeengt. Der Rückstand wird mit Petroläther kristallisiert.

Ausb. 111,45 g (64%).
Schmp. 73—76°, $[\alpha]_D^{25} = -28,7°$ (c = 1, in Methanol).

$C_{23}H_{26}NO_6Cl_3$ (518,84) Ber. C 53,24 H 5,05 N 2,70
Gef. C 53,5 H 5,2 N 2,9

Zu einer Lösung von 30,5 g (150 mmol) H-Glu(OBu$^t$)-OH und 20,25 g (130 mmol) HOBt in 200 ml Dimethylformamid gibt man 19,2 ml (150 mmol) H-Äthylmorpholin und 77,8 g Ddz-Val-OTcp. Man läßt 4 Stunden bei Raumtemperatur rühren. Ist im DC kein H-Glu(OBu$^t$)-OH mehr nachweisbar wird, im Hochvakuum eingeengt. (Andernfalls bringt man durch Zusatz von wenig Ddz-Val-OTcp die Reaktion zu Ende). Das resultierende Öl wird zwischen 300 ml Essigester und 300 ml Wasser verteilt. Die Essigesterphase wird zwei mal mit je 150 ml Citratpuffer (pH 3) und ein mal mit 150 ml Wasser ausgeschüttelt, über $Na_2SO_4$ getrocknet und eingeengt.

Ausb. 138,7 g (78,7 g = 100%). Das resultierende Öl wird ohne weitere Reinigung und Charakterisierung als 150 mmol weiter umgesetzt.

$C_{26}H_{40}N_2O_9$ (524,6)

*3. Ddz-Val-Glu(OBu$^t$)-Gln-ONb*

Zu einer Lösung von rohem, verunreinigtem 138,7 g (= 150 mmol) Ddz-Val-Glu(OBu$^t$)-OH, 47,65 g (150 mmol) H-Gln-ONb HCl und 20,25 g (150 mmol) HOBt in 450 ml Dimethylformamid gibt man bei 0°C 19,2 ml (150 mmol) N-Äthylmorpholin und 33 g (160 mmol) DCC. Man läßt 1 Stunde bei 0°C und 4 Stunden bei Raumtemperatur rühren. Man läßt über Nacht bei Raumtemperatur stehen, saugt den Niederschlag ab und engt das Filtrat im Hochvakuum ein. Das resultierende Öl wird zwischen 300 ml Essigester und 300 ml Wasser verteilt. Die Essigesterphase wird nacheinander mit gesättigter $NaHCO_3$-Lösung, Citratpuffer (pH 3) gesättigter $NaHCO_3$-Lösung und Wasser ausgeschüttelt, über $Na_2SO_4$ getrocknet und eingeengt. Das Öl kristallisiert mit Äther. Mit Äther wird gewaschen.

Ausb. 110,95 g (94%).
Schmp 135—138°, 138—142°, $[\alpha]_D^{22} = -49,4°$ (c = 1, in Methanol).

$C_{38}H_{53}N_5O_{13}$ (787,9) Ber. C 57,93 H 6,78 N 8,89
Gef. C 57,8 H 7,0 N 9,2

*4. H-Val-Glu(OBu$^t$)-Gln-ONb-trifluoracetat*

39,4 g (50 mmol) Ddz-Val-Glu(OBu$^t$)-Gln-ONb werden in einer Mischung aus 875 ml einer 5%igen Trifluoressigsäure-Methylenchlorid-Lösung (mit 1% Wasser) (= 500 mmol Trifluoressigsäure) uns 87,5 ml Anisol gelöst. Man läßt 30 Minuten bei Raumtemperatur stehen, neutralisiert mit 50 ml Pyridin und engt ein. Im Hochvakuum wird nachgetrocknet. Der Rückstand wird zwischen Essigester und Wasser verteilt. Die Essigesterphase wird noch zwei mal mit wenig Wasser ausgeschüttelt, über $Na_2SO_4$ getrocknet und eingeengt. Der Rückstand wird mit Äther verrieben und im Hochvakuum getrocknet.

Ausb. 23,6 g amorphe Substanz (69%).
$[\alpha]_D^{24} = -5,3°$ (c = 1, in Methanol).

$C_{26}H_{39}N_5O_9 \cdot CF_3COOH$ (679,69) Ber. C 49,48 H 5,93 N 10,30
Gef. C 50,0. H 5,8 N 10,1

*5. Ddz-Gly-OTcp*

Zu einer Lösung von 23,8 g (ca. 80 mmol) Ddz-Gly-OH (Liebigs Ann. 763, 162—172 (1972)) und 16 g (ca. 80 mmol) 2,4,5-Trichlorphenol in 150 ml Essigester gibt man bei 0°C eine Lösung von 16,5 g (80 mmol) DCC in 50 ml Essigester. Man läßt über Nacht bei Raumtemperatur stehen, saugt den Niederschlag ab und engt das Filtrat ein. Der Rückstand wird mit Petroläther kristalliert. Man läßt 6 Stunden im Kühlraum stehen. Der Niederschlag wird abgesaugt und mit Petroläther gewaschen.

Ausb. 36,3 g (95%).
Schmp. (111)—115°.

$C_{20}H_{20}NO_6Cl_3$ (476,76) Ber. C 50,39 H 4,23 N 2,94
Gef. C 50,8 H 4,3 N 2,95

*6. Ddz-Gly-Ile-OMe*

Zu einer Lösung von 14,54 g (80 mmol) H-Ile-OMe·HCl und 10,8 g (80 mmol) HOBt in 50 ml Dimethylformamid gibt man bei −3°C (10,3 ml (80 mmol) N-Äthylmorpholin und bei 0°C 40 g (84 mmol) Ddz-Gly-OTcp. Man läßt 3 Stunden bei Raumtemperatur rühren, und läßt über Nacht stehen. (Im DC war andertags noch etwas H-Ile-OMe feststellbar. Es wurden daher nochmals 2,86 g (6 mmol) Ddz-Gly-OTcp zugegeben und gerührt. Nach 5 Stunden war kein H-Ile-OMe feststellbar.) Die im DC von H-Ile-OMe freie Lösung wird am Hochvakuum eingeengt und das resultierende Öl zwischen Essigester und Wasser verteilt. Die Essigesterlösung wird nacheinander mit gesättigter $NaHCO_3$-Lösung, Citratpuffer (pH 3), $NaHCO_3$-Lösung und Wasser gewaschen und über $Na_2SO_4$ getrocknet. Die Essigesterlösung wird über 400 g basisches $Al_2O_3$ filtriert, das Eluat eingeengt und am Hochvakuum getrocknet.

Ausb. 30,6 zähes Öl (90%).
$[\alpha]_D^{25} = -6,8°$ (c = 1, in Methanol).

$C_{21}H_{32}N_2O_7$ (424,5) Ber. C 59,42 H 7,60 N 6,60
Gef. C 59,1 H 7,7 N 6,3

*7. Ddz-Gly-Ile-OH*

10 g (23,56 mmol) Ddz-Gly-Ile-OMe werden in einer Mischung aus 24 ml Dioxan und 8 ml Wasser gelöst und gegen Thymolphthalein als Indikator mit 1N NaOH tritriert. Verbrauch je nach Essigestergehalt mehr als 23 ml. Nach Neutralisation mit Zitronensäure wird das Dioxan abdestilliert. Der Rückstand wird in Wasser aufgenommen und bei 0°C mit 2N Zitronensäure auf pH 3 gestellt. Das ausfallende Öl wird mit Essigester extrahiert. Die wässrige Phase wird, wenn nötig nochmals auf pH 3 angesäuert und mit Essigester extrahiert. Die vereinigten Essigesterextrakte werden mit Wasser ein mal ausgeschüttelt, über $Na_2SO_4$ getrocknet und eingeengt. Im Hochvakuum wird getrocknet. Die Substanz wird amorph.

Ausb. 8,9 g (92%).
Schmp. 80—90°, $[\alpha]_D^{23} = +4{,}2°$ (c = 1, in Methanol).

$C_{20}H_{30}N_2O_7$ (410,48) Ber. C 58,52 H 7,37 N 6,83
Gef. C 58,8 H 7,5 N 6,5

*8. Ddz-Gly-Ile-Val-Glu(OBut)-Gln-ONb*

Zu einer Lösung von 15,8 g (38,5 mmol) Ddz-Gly-Ile-OH, 23,8 g (35 mmol) H-Val-Glu(OBu$^t$)-Gln-ONb-trifluoracetate und 5,2 g (38,5 mmol) HOBt in 60 ml Dimethylformamid gibt man bei 0°C 4,48 ml (35 mmol) N-Äthylmorpholin und 8,47 g (41 mmol) DCC. Man läßt eine Stunde bei 0°C, mehrere Stunden bei Raumtemperatur rühren und über Nacht bei Raumtemperatur stehen. Es bildet sich ein Gelee, das in gekühltes $NaHCO_3$-Lösung eingerührt wird. Das Produkt wird abgesaugt und nacheinander mit Citratpuffer (pH 3), $NaHCO_3$-Lösung und Wasser verrieben und jeweils abgesaugt. Über $P_2O_5$ wird getrocknet.

Ausb. 40,3 g.
Zur weiteren Reinigung wird aus 500 ml Äthanol umkristallisiert.
Ausb. 21,45 g (64%).
Schmp. 205—207° $[\alpha]_D^{25} = -12{,}3°$ (c = 1, in Dimethylformamid).

$C_{46}H_{67}N_7O_{15}$(958,1) Ber. C 57,67 H 7,05 N 10,23
Gef. C 57,6 H 7,2 N 10,0

*9. Ddz-Gly-Ile-Val-Glu(OBu$^t$)-Gln-OH*

19,16 g (20 mmol) Ddz-Gly-Ile-Val-Glu(OBu$^t$)-Gln-ONb werden in einer Mischung von 100 ml Dimethylformamid und 100 ml Methanol gelöst. Nach Zugabe von Pd/$BaSO_4$ wird katalytisch hydriert. Nach 6 Stunden war alles hydriert. Die Suspension wird auf ca. 70°C erhitzt und in der Wärme vom Katalysator abgesaugt. Mit warmen Dimethylformamid wird nachgewaschen. Das Filtrat wird eingeengt unde der Rückstand mit 100 ml Methanol verrieben. Man läßt über nacht im Kühlraum stehen, saugt ab und wäscht mit kaltem Methanol nach. Trocknen im Vakuum über $P_2O_5$.

Ausb. 14 g (85%),
Schmp. 238—241°(Zers.) $[\alpha]_D^{29} = -13{,}3°$ (c = 1, in Dimethylformamid)

$C_{39}H_{62}N_6O_{13}$ (822, 97) Ber. C 56, 92 H 7,59 N 10,21
Gef. C 56,0 H 7,6 N 10,8

*VI. Fragmentkondensationen*

*1. Ddz-Leu-Glu(Obu$^t$)-Asn-Tyr(Bu$^t$)-Cys(Trt)-Asn-OBu$^t$*

Zu einer auf 0°C abgekühlten Lösung von 26,4 g (30 mMol) Ddz-Leu-Glu(OBu$^t$)-Asn-Tyr(Bu$^t$)-OH-hemihydrat, 16,0 g (30 mMol) H-Cys(Trt)-Asn-OBu$^t$ und 4,05 g (30 mMol) HOBt in 60 ml Dimethylformamid gibt man eine auf 0°C abgekühlte Lösung von 6,6 g DCC in 20 ml Dimethylformamid. Man läßt den Ansatz 2 Stunden bei 0°C und über Nacht bei Raumtemperatur rühren. Der Dicyclohexylharnstoff wird abgesaugt und mit 20 ml Dimethylformamid gewaschen. Das Filtrat gießt man unter Rühmen vorsichtig in ca. 350 ml gekühlte $NaHCO_3$-Lösung und extrahiert mit 600 ml Essigester.

Die Essigesterphase wird nacheinander 1 × mit 300 ml $NaHCO_3$-Lösung, 1 × mit 300 ml Citratpuffer (pH 4) und 1 × mit 100 ml $NaHCO_3$-Lösung ausgeschüttelt, über $Na_2SO_4$ getrocknet und eingeengt. Der Rückstand wird mit Äther verrieben.

Ausb. 28—34 g (67—81%).
Schmp 161—165°, $[\alpha]_D^{25} = -13{,}1°$ (c = 1, in $CH_2Cl_2$).

$C_{74}H_{98}N_8O_{16}S$ (1387,73) Ber. C 64,05 H 7,12 N 8,07 S 2,31
Gef. C 63,8 H 7,3 N 8,0 S 2,7

*2. H-Leu-Glu(OBu^t)-Asn-Tyr(Bu^t)-Cys(Trt)-Asu-OBu^t-trifluoracetat*

27,75 g (20 mmol) Ddz-Leu-Glu(OBu$^t$)-Asn-Tyr(Bu$^t$)-Cys(Trt)-Asn-OBu$^t$ werden bei Raumtemperatur in einer Mischung aus 17,5 ml Trifluoressigsäure (0,2 mol), 3,5 ml Wasser und 330 ml Methylenchlorid (= ca. 350 ml 5%ige Trifluoressigsäure mit 1% Wasser in Methylenchlorid) eingetragen. Man läßt 2 Stunden bei Raumtemperatur rühren. Darauf kühlt man auf 0°C ab, neutralisiert die Trifluoressigsäure mit 17,75 ml (220 mmol) Pyridin und engt im Vakuum ein. Der Rückstand wird in 250 ml Essigester gelöst und 3 mal mit 25 ml Wasser ausgeschüttelt. Die Essigesterphase wird *ohne* zuvor getrocknet zu werden, eingeengt. Der Rückstand wird im Hochvakuum getrocknet und mit Äther verrieben. (Statt Äther kann auch Diisopropyläther verwendet werden.) Der Niederschlag wird abgesaugt, mit Äther gewaschen und getrocknet. Ausb. 25,2 g.

Zur weiteren Reinigung werden ca. 6 g-Portionen in 30 ml Essigester zum Sieden erhitzt und warm zentrifugiert. Die Niederschläge werden je 2 mal mit Äther verrührt, zentrifugiert und am Hochvakuum über $P_2O_5$ getrocknet.

Ausb. 22,3 g (86%).
Schmp. 178—182°, 161—164°, 166—171°, $[\alpha]_D^{22}$ = +3,5 bis + 4,0° (c = 1 in 90-proz. Essigsäure).

| | | |
|---|---|---|
| $C_{64}H_{85}N_8O_{14}SF_3$ (1279,5) | Ber. | C 60,08 H 6,70 N 8,76 S 2,51 |
| | Gef. | C 59,4 H 6,5 N 9,1 S 2,9 |

| Aminosäureanalyse: | Asp | Glu | Cys | Leu | Tyr |
|---|---|---|---|---|---|
| | 2,0 | 1,05 | 0,87 | 0,99 | 1,05 |

*3. Ddz-Tyr(Bu^t)-Gln-Leu-Glu(OBu^t)-Asn-Tyr(Bu^t)-Cys(Trt)-Asn-OBu^t*

9,7 g (16,5 mmol) Ddz-Tyr(Bu$^t$)-Gln-OH, 19,2 g (15 mmol) H-Leu-Glu(OBu$^t$)-Asn-Tyr(Bu$^t$)-Cys(Trt)-Asn-OBu$^t$. $CF_3COOH$ und 2,025 g (15 mmol) HOBt werden in 30 ml Dimethylformamid unter Rühren bei Raumtemperatur gelöst. Man kühlt dann auf 0°C ab und versetzt mit 1,95 ml (15 mmol) N-Äthylmorpholin und einer Lösung von 3,3 g (16 mmol) DCC in 9 ml Dimethylformamid. Man läßt 1 Stunde bei 0°C und 4 Stunden bei Raumtemperatur rühren, läßt über Nacht bei Raumtemperatur stehen und saugt den Dicyclohexylharnstoff ab. Mit je 4,5 ml Dimethylformamid wird zwei mal nachgewaschen. Das Filtrat läßt man unter Rühren zu 159 ml gesättigter $NaHCO_3$-Lösung einfließen und rührt so lange bis ein pulvriger Niederschlag entsteht. Der Niederschlag wird abgesaugt, mit Citratpuffer (pH 3) verrieben, abgesaugt und mit Wasser neutral gewaschen. Der Niederschlag wird im Hochvakuum getrocknet. Ausb. 23,1 g.

Die Rohsubstanz wird auf dem Dampfbad bis fast zum Sieden erwärmt. Die dünne Suspension wird über Nacht in den Kühlraum gestellt und der Niederschlag abgesaugt. Mit Essigester und Äther wird gewaschen.

Ausb. 20 g (76,8%).
$[\alpha]_D^{24}$ = −10,2° (c = 1, in Methanol).

Die Substanz zersetzt sich ab 205° und verkohlt bei ca. 250°.

$C_{92}H_{123}N_{11}O_{20}S$ (1735,15)

| Aminosäureanalyse: | Asp | Glu | Cys | Leu | Tyr |
|---|---|---|---|---|---|
| Ber: | 2,0 | 2,0 | 1,0 | 1,0 | 2,0 |
| Gef: | 2,00 | 2,01 | 0,75 | 0,99 | 1,95 |

*4. H-Tyr(Bu^t)-Gln-Leu-Glu(OBu^t)-Asn-Tyr(Bu^t)-Cys(Trt)-Asn-OBu^t-trifluoracetat*

3,5 g (2 mmol) Ddz-Tyr(Bu$^t$)-Gln-Leu-Glu(OBu$^t$)-Asn-Tyr(Bu$^t$)-Cys(Trt)-Asn-OBu$^t$ werden in einer Mischung aus 1,75 ml Trifluoressigsäure (20 mmol), 0,35 ml Wasser und 33 ml Methylenchlorid (= ca. 35 ml einer 5%igen Trifluoressigsäurelösung mit 1% Wasser) und 3,5 ml Anisol unter Rühren gelöst. Man läßt 3 Stunden bei Raumtemperatur rühren, versetzt anschließend mit 2 ml (24,8 mmol) Pyridin und engt im Hochvakuum ein. Der Rückstand wird mit Äther verrieben, abgesaugt und mit Äther gewaschen und getrocknet. Danach wird die Substanz mit Wasser verrieben, abgesaugt, mit Wasser gewaschen und über $P_2O_5$ getrocknet. Ausb. 3,35 g.

Zur weiteren Reinigung wurde die Substanz mit je 20 ml Essigester kurz zum Sieden erhitzt und heiß abgesaugt. Mit Äther wurde nachgewaschen.

Ausb. 3,0 g (92%).
Schmp. 255—265° (Zers.), $[\alpha]_D^{24}$ = −23,8° (c = 1, in Methanol).

$C_{80}H_{109}N_{11}O_{16}S \cdot CF_3COOH$ (1626,9)

| Aminosäureanalyse: | Asp | Glu | Cys | Leu | Tyr |
|---|---|---|---|---|---|
| Ber: | 2 | 2 | 1 | 1 | 2 |
| Gef: | 1,95 | 2,08 | 0,81 | 1,07 | 2,04 |

*5. Ddz-Ile-Cys(Trt)-Ser-Leu-Tyr(Bu^t)-Gln-Leu-Glu(OBu^t)-Asn-Tyr(Bu^t)-Cys(Trt)-Asn-OBu^t*

Zu einer Lösung von 22,8 g (14 mmol) H-Tyr(Bu$^t$)-Gln-Leu-Glu(OBu$^t$)-Asn-Tyr(Bu$^t$)-Cys(Trt)-Asn-OBu$^t$, 14,1 g (15,4 mmol) Ddz-Ile-Cys(Trt)-Ser-Leu-OH.1 $H_2O$ und 2,08 g (15,4 mmol) HOBt und 1,8 ml (14 mmol) N-Äthylmorpholin in 70 ml Dimethylformamid gibt man bei 0°C 3,4 g (16,4 mmol) DCCl. Man läßt 1 Stunde bei 0°C und 3 Stunden bei Raumtemperatur rühren, läßt über Nacht bei Raumtemperatur stehen und saugt den Niederschlag ab. Das Filtrat wird im Vakuum eingeengt und der Rückstand nacheinander mit $NaHCO_3$-Lösung, Citratpuffer (pH 3), $NaHCO_3$-Lösung und Wasser verrieben und jeweils abgesaugt. Über $P_2O_5$ wird getrocknet.

Ausb. 33,18 g (99%).

Zur weiteren Reinigung wird die trockene Substanz in 300 ml warmen Essigester suspendiert und kurz auf dem Dampfbad erwärmt. Nach Zugabe von 300 ml Äther wird noch warm abgesaugt. Mit Äther wird nachgewaschen und am Hochvakuum getrocknet.

Ausb. 28,1 g (84%).
Schmp. 265—270° (Zers.), $[\alpha]_D^{22} = -20,3°$ (c = 1, in Dimethylformamid).

Anschließend wurde noch mit 100 ml Methanol erhitzt und nach Stehen im Kühlraum (über Nacht) abgesaugt und getrocknet.

Ausb. 23,75 g (71%).
$[\alpha]_D^{22} = -25,4°$ (c = 1 in Dimethylformamid).

$C_{129}H_{169}N_{15}O_{25}S_2$ (2394,0)

| Aminosäureanalyse: | Asp | Ser | Glu | Cys | Ile | Leu | Tyr |
|---|---|---|---|---|---|---|---|
| Ber: | 2 | 1 | 2 | 2 | 1 | 2 | 2 |
| Gef: | 2,10 | 0,95 | 1,77 | 0,49 | 0,83 | 2 | |

*6. H-Ile-Cys(Trt)-Ser-Leu-Tyr(Bu^t)-Gln-Leu-Glu(OBu^t)-Asn-Tyr(Bu^t)-Cys(Trt)-Asn-OBut-trifluoracetat*

24 g (ca. 10 mmol) Ddz-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$ werden in einer Mischung aus 8,75 ml (100 mmol) Trifluoressigsäure, 1,75 ml Wasser und 165 ml Methylenchlorid (= ca. 175 ml einer 5%igen Trifluoressigsäure-Methylenchlorid-Lösung mit 1% Wasser) und 17,5 ml Anisol unter Rühren gelöst. Man läßt 4 Stunden bei Raumtemperatur rühren. Danach versetzt man mit 10 ml (124 mmol) Pyridin und engt ein. Der Rückstand wird mit Äther verrieben, abgesaugt und mit Äther gewaschen und im Vakuum getrocknet. Danach wird die Substanz mit Wasser verrieben, abgesaugt, mit Wasser gewaschen und über $P_2O_5$ getrocknet.

Zur weiteren Reinigung wird die Substanz 2 mal mit je 100 ml Essigester ausgekocht und jeweils heiß abgesaugt.

Ausb. 22,7 g (99%).
Schmp. 234° (Zers.), $[\alpha]_D^{26} = -11,7°$ (c = 1, in Dimethylformamid).

$C_{117}H_{155}N_{15}O_{21}S_2 \cdot CF_3COOH$ (2285,8)

| Aminosäureanalyse: | Asp | Ser | Glu | Cys | Ile | Leu | Tyr |
|---|---|---|---|---|---|---|---|
| Ber: | 2 | 1 | 2 | 2 | 1 | 2 | 2 |
| Gef: | 2,05 | 0,99 | 1,88 | 0,43 | 0,73 | 2,12 | 2,05 |

*7. Ddz-Cys(Trt)-Cys(Trt)-Thr(Bu^t)-Ser(Bu^t)-Ile-Cys(Trt)-Ser-Leu-Tyr(Bu^t)-Gln-Leu-Glu(OBu^t)-Asn-Tyr(Bu^t)-Cys(Trt)-Asn-OBu^t*

Zu einer Lösung von 16 g (7 mmol) H-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$-trifluoracetat, 9,5 g (7,7 mmol) Ddz-Cys(Trt)-Cys(Trt)-Thr(Bu$^t$)-Ser(Bu$^t$)-OH, 1,04 g (7,7 mmol) HOBt und 0,9 ml (7 mmol) N-Athylmorpholin in 70 ml einer Dimethylformamid/Dimethylacetamid-Mischung (1:1) gibt man bei 0°C 1,7 g (8,25 mmol) DCC. Man läßt 2 Stunden bei 0°C und anschließend bei Raumtemperatur rühren. Man läßt über Nacht Stehen und rührt anderntags noch einmal 5 Stunden bei Raumtemperatur. Der ausgefallene DC-Harnstoff wird abgesaugt. Das Filtrat wird auf die Hälte am Hochvakuum eingeengt und anschließend unter Rühren in Eiswasser eingetropft. Der Niederschlag wird abgesaugt und nacheinander mit $NaHCO_3$-Lösung, Zitratpuffer (pH 3), $NaHCO_3$-Lösung und Wasser gewaschen und über $P_2O_5$ getrocknet. Zur weiteren Renigung wird mit 100 ml Essigester unter Erwärmen verrührt. Nach Zugabe von 200 ml Äther wird der Niederschlag noch warm abgesaugt und getrocknet. Anscließend wird die Substanz mit 220 ml 50-proz. wässrigem Methanol und zum Schluß mit 200 ml Methanol erhitzt und jeweils heiß abgesaugt.

Ausb. 19,42 g (81%).
Schmp. 250—260° (Zers.), $[\alpha]_D^{22} = -14{,}2°$ (c = 1, in Dimethylformamid).

$C_{188}H_{235}N_{19}O_{31}S_4 \cdot 2\ H_2O$ (3421,4) Ber. C 66,00 H 7,04 N 7,78 S 3,75
Gef. C 65,4 H 7,1 N 8,0 S 3,8

Aminosäureanalyse (nach Oxydation von Cystein zur Cysteinsäure):

| | Asp | Thr | Ser | Glu | Cys | Ile | Leu | Tyr |
|---|---|---|---|---|---|---|---|---|
| Ber. | 2 | 1 | 2 | 2 | 4 | 1 | 2 | 2 |
| Gef. | 1,99 | 0,89 | 1,69 | 1,92 | 3,97 | 0,87 | 2,07 | — |

Tyrosin fehlt, da es bei der Oxydation zerstört wird.

Aminosäureanalyse ohne Oxydation:

| | | | | | | | | |
|---|---|---|---|---|---|---|---|---|
| Gef. | 2,08 | 0,92 | 1,87 | 2,00 | 1,55 | 0,88 | 2,00 | 1,92 |

*8. H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$-trifluoracetat*

10,26 g (3 mmol) Ddz-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$ werden in einer Mischung aus 5,25 ml (60 mmol) Trifluoressigsäure, 1,05 ml Wasser und 99 ml Methylenchlorid (= ca. 105 ml einer 5-proz. Trifluoressigsäure-Methylenchlorid-Lösung mit 1% Wasser) und 10,5 ml Anisol unter Rühren gelöst. Man läßt 4 Stunden bei Raumtemperatur rühren. Danach versetzt man mit 10,5 ml Pyridin und engt ein. Im Hochvakuum wird nachdestilliert. Der ölige Rückstand wird mit Äther verrieben und der Niederschlag abgesaugt und getrocknet. Anschließend wird die Substanz mit Wasser gewaschen und über $P_2O_5$ getrocknet. Zur weiteren Reinigung wird die Substanz drei mal mit je 100 ml Essigester aufgekocht und jeweils heiß abgesaugt.

Ausb. 7,96 g (81%).
Schmp. 240—245° (Zers.), $[\alpha]_D^{23} = -10{,}0°$ (c = 1, in Dimethylformamid).

$C_{176}H_{221}N_{19}O_{27}S_4 \cdot CF_3COOH$ (3277,15)

Aminosäureanalyse:

| | Asp | Thr | Ser | Glu | Cys | Ile | Leu | Tyr |
|---|---|---|---|---|---|---|---|---|
| Ber. | 2 | 1 | 2 | 2 | 4 | 1 | 2 | 2 |
| Gef. | 1,96 | 0,80 | 1,49 | 2,03 | 3,21 | 1,10 | 2,04 | 1,78 |

*9. Ddz-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$*

Zu einer Lösung von 3,28 g (1 mmol) H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$-trifluoracetat, 1 g (ca. 1,2 mmol) Ddz-Gly-Ile-Val-Glu(OBu$^t$)-Glu-OH und 162 mg HOBt (1,2 mmol) in 20 ml einer Mischung aus Dimethylformamid und Dimethylacetamid (1:1) gibt man 0,13 ml N-Äthylmorpholin und bei 0°C 310 mg (1,5 mmol) DCC. Man läßt 1 Stunde bei 0°C und 6 Stunden bei Raumtemperatur rühren und läßt über Nacht stehen. Es entsteht ein dickes Gelee, das mit Eiswasser versetzt wird. Der sich bildende Niederschlag wird abgesaugt und nacheinander mit $NaHCO_3$-Lösung, Citratpuffer (pH 3), $NaHCO_3$-Lösung und Wasser verrieben und jeweils abgesaugt. Zur weiteren Reinigung wird 2 mal mit je 25 ml Essigester ausgekocht und heiß abgesaugt.

Ausb. 2,87 g (72%).
Schmp. 260—278° (Zers.), $[\alpha]_D^{24} = -24{,}5°$ (c = 1, in Dimethylformamid).

$C_{215}H_{281}N_{25}O_{39}S_4$ (3968,07)

Aminosäureanalyse:

| | Asp | Thr | Ser | Glu | Gly | Cys | Val | Ile | Leu | Tyr |
|---|---|---|---|---|---|---|---|---|---|---|
| Ber. | 2 | 1 | 2 | 4 | 1 | 4 | 1 | 2 | 2 | 2 |
| Gef. | 2,06 | 0,88 | 2,04 | 3,97 | 1,01 | 2,59 | 0,60 | 1,45 | 1,93 | 1,93 |

*10. H-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$-trifluoracetat*

2,78 g (0,7 mmol) Ddz-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$ werden mit 2,5 ml Anisol in 25 ml einer 5-proz. Trifluoressigsäure-Methylenchlorid-Lösung mit 1% Wasser (= ca. 14 mmol Trifluoressigsäure) 4 Stunden bei Raumtemperatur gerührt. Danach gibt man 1,4 ml Pyridin zu und engt zuerst am Wasserstrahlpumpenvakuum und zum Schluß am ' ɔchvakuum ein. Der Rückstand wird mit Äther verrieben, abgesaugt und mit Äther gewaschen und getrocknet. Die Substanz wird anschließend mit Wasser verrieben, abgesaugt, mit Wasser gewaschen und über $P_2O_5$ getrocknet. Zur weiteren Reinigung wird zwei mal mit je 30 ml Essigester aufgekocht und heiß abgesaugt.

Ausb. 1,85 g (68,5%).
Schmp. 275—280° (Zers.), $[\alpha]_D^{23} = -24,0°$ (c = 1, in Dimethylformamid).

$$C_{203}H_{267}N_{25}O_{35}S_4 \cdot CF_3COOH \ (3859,8)$$

Aminosäureanalyse:

| | Asp | Thr | Ser | Glu | Gly | Cys | Val | Ile | Leu | Tyr |
|---|---|---|---|---|---|---|---|---|---|---|
| Ber. | 2 | 1 | 2 | 4 | 1 | 4 | 1 | 2 | 2 | 2 |
| Gef. | 2,05 | 1,08 | 1,80 | 4,00 | 0,97 | 2,40 | 0,71 | 1,51 | 2,06 | 1,88 |

**Patentansprüche**

1. Peptid der Formel I

H-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

2. Verfahren zur Herstellung des Peptides der Formel I, das dadurch gekennzeichnet, daß man das Peptid der Formel II

H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

mit dem Peptid der Formel III

X-Gly-Ile-Val-Glu(OBu$^t$)-Gln-OH

worin X eine Aminoschutzgruppe bedeutet, die durch 5% Trifluoressigsäure in 95% Methylenchlorid abspaltbar ist, nach der Dicyclohexylcarbodiimid-Methode unter Zusatz von n-Hydroxyverbindungen kondensiert und die N-terminale Schutzgruppe X aus dem erhaltenen Peptid selektiv mit etwa 5%iger Trifluoressigsäure in Methylenchlorid abspaltet.

**Revendications**

1. Peptide de formule I:

H-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

2. Procédé de préparation du peptide de formule I, caractérisé en ce qu'on condense, selon le procédé au dicyclohexylcarbodiimide avec addition de composés N-hydroxy, le peptide de formule II:

H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

avec le peptide de formule III

X-Gly-Ile-Val-Glu(OBu$^t$)-Gln-OH

dans laquelle X représente un groupe amino-protecteur qui peut être éliminé par de l'acide trifluoroacétique à 5% dans 95% de chlorure de méthylène, puis on élimine sélectivement le groupe protecteur N-terminal X du peptide obtenu avec de l'acide trifluoroacétique à environ 5% dans le chlorure de méthylène.

## Claims

1. The peptide of the formula I

H-Gly-Ile-Val-Glu(OBu$^t$)-Gln-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

2. A process for the preparation of the peptide of the formula I which comprises condensing the peptide of the formula II

H-Cys(Trt)-Cys(Trt)-Thr(Bu$^t$)-Ser(Bu$^t$)-Ile-Cys(Trt)-Ser-Leu-Tyr(Bu$^t$)-Gln-Leu-Glu(OBu$^t$)-Asn-Tyr(Bu$^t$)-Cys(Trt)-Asn-OBu$^t$

by the dicyclohexylcarbodiimide method with the addition of N-hydroxy compounds with the peptide of the formula III

X-Gly-Ile-Val-Glu(OBu$^t$)-Gln-OH

in which X is an amino protective group capable of being split off by 5% trifluoroacetic acid in 95% of methylene chloride and selectively splitting off the N-terminal protective group X from the peptide obtained with about 5% trifluoroacetic acid in methylene chloride.